# EUROPEAN PATENT SPECIFICATION

(11) **EP 4 312 936 B1**
(45) Date of publication and mention of the grant of the patent: **18.02.2026**
(21) Application number: 22829513.5
(22) Date of filing: 23.06.2022
(51) Int. Cl.: A61H 1/00

(54) **DIRECTIONAL FLOW COMPRESSION GARMENT**
KOMPRESSIONSKLEIDUNG MIT GERICHTETEM FLUSS
VÊTEMENT DE COMPRESSION À FLUX DIRECTIONNEL

(30) Priority: 24.06.2021 US 202163214619 P
(43) Date of publication of application: 07.02.2024
(73) Proprietor: Koya Medical, Inc., Oakland, CA 94607 (US)
(72) Inventor: PAMPLIN, John C., Oakland, California 94607 (US); MEEHAN, Connor, Oakland, California 94607 (US); BALDWIN, Jarren, Oakland, California 94607 (US); DORAISWAMY, Anand, Oakland, California 94607 (US)
(74) Representative: Boult Wade Tennant LLP
(86) International application number: PCT/US2022/073117
(87) International publication number: WO 2022/272285

(56) References cited:
- US-A1- 2003 045 821
- US-A1- 2010 312 160
- US-A1- 2010 312 160
- US-A1- 2015 133 836
- US-A1- 2020 000 676
- US-A1- 2020 000 676
- US-B1- 6 656 141
- US-B2- 10 893 968
- US-B2- 7 135 007
- US-B2- 8 801 643
- US-B2- 9 421 142

## Description

### FIELD

This disclosure relates to compression garments, specifically to compression garments to promote lymphatic circulation.

### BACKGROUND

Lymphedema is a medical condition in which excess fluid, such as lymph fluid, collects in the body. In some instances, this excess fluid may collect in a specific area of the body, such as a limb, and cause swelling (i.e., edema). Left untreated, numerous additional symptoms may occur such as infections, fatigue, restricted range of motion, hardening of the skin/tissue, etc.

US 2003/045821 A1 describes an apparatus for treating an elevated concentration of interstitial fluid in a body area of a patient includes a pad constructed and arranged to be placed in pressure-applying, conforming relation to the body area. The pad includes inner and outer liners connected at marginal edges thereof to one another, and a foam pad having a multiplicity of resilient protrusions is encased between the inner and outer liners. A plurality of channels are formed in areas between the protrusions by securing the inner liner in close proximity to the outer liner along each channel. The channels and protrusions are constructed and arranged such that when the pad is placed in pressure-applying, conforming relation to the body area, localized pressure generated on the body area at each protrusion is greater than localized pressure generated on the body area along the channel. The pad can be rolled into a garment, such as a sleeve, a vest, or a brief, and worn by the patient over the afflicted body area, for example, a limb or the pelvic area. Fluid redistribution is achieved by the combination of pressure applied at the protrusions and low pressure areas between protrusions and along the channels, which provide pathways along which the excess interstitial fluid may flow out of the afflicted body area.

US 6,656,141 B1 describes an apparatus for applying pressure to a body part comprising: a first sleeve comprising a multiplicity of pressure-applying resilient protrusions configured to apply a therapeutic pressure to a surface of the body part of a patient; and a second sleeve slidably received over the first sleeve, the second sleeve configured to apply a pressure to the first sleeve, thereby increasing the therapeutic pressure applied by the first sleeve on the limb.

US 7,135,007 B1 describes a compression garment for compressing a portion of a body of a patient includes a tubular body. The body includes an outer layer and an inner layer secured to the outer layer. The inner layer at least partially bounds a channel adapted to receive a portion of a body of a patient. The inner layer includes a backing having a plurality of pressure projections extending therefrom. A layer of compressible cushioning material is disposed between the outer layer and the inner layer. Compression straps are disposed on the body for selectively constricting the body.

### SUMMARY OF THE INVENTION

The presently claimed invention is defined by the appended claims.

### SUMMARY OF THE DISCLOSURE

Lymphedema may be treated by promoting the drainage of fluid (e.g., lymph) from a region of the body. Disclosed herein are garments and related components (e.g., flow directing units) that promote the movement of fluid from an area of the body. For example, a garment, such as a sleeve, may be worn on a limb (e.g., arm, leg, hand, foot, etc.) of a user or any other portion of the body (e.g., torso, head, neck, etc.). The garment can include one or more flow directing units, which can also be referred to as garment inserts. The one or more flow directing units can include an elongate wall with a plurality of teeth extending therefrom. The plurality of teeth may extend at an angle relative to the elongate wall. The garment can be worn by a user to orient ends of the plurality of teeth toward the skin of the user. When worn, the plurality of teeth can provide a directional force when compressed to urge/promote fluid flow. When worn, a compressive force applied to the outside of the garment can be translated by the plurality of teeth to urge fluid disposed inside the user to flow in a predetermined direction based on the orientation of the plurality of teeth. In some instances, the garment can be worn by the user while the user sleeps such that the user's weight against a surface (e.g., bed) provides the compressive forces that are translated by the garment to urge fluid flow. The garment can include a bioimpedance sensing device to sense characteristics, such as volume, of the portion of the body wearing by the garment and/or a vibration unit to promote flow of fluid within the body.

In one embodiment, a garment configured to be worn by a user to promote lymphatic flow comprises: a plurality of flow directing units, each of the plurality of flow directing units comprising a wall and a plurality of teeth extending from the wall at an angle, the plurality of teeth configured to face skin of the user when the garment is worn; wherein the plurality of teeth are configured to translate a compressive force applied to the garment to promote fluid within the user to flow in a direction.

The plurality of teeth may in a repeating pattern, in a repeating saw-toothed pattern, and/or evenly distributed along the wall. The angle is acute. The plurality of teeth may include rounded ends. The plurality of teeth may have a thickness that tapers to an end. The plurality of teeth may have a thickness that is consistent. The plurality of flow directing units may be made of a low durometer material. The plurality of flow directing units may be made of foam. The plurality of flow directing units may be made of plastic. The plurality of flow directing units may be made of rubber.

The garment may include channels configured to receive the plurality of flow directing units to maintain an orientation of the plurality of flow directing units. The plurality of flow directing units may be sewn into the garment. The garment may be configured to be worn on an arm, leg, foot, hand, shoulder, trunk, head, and/or neck of the user. The garment may be configured to position the plurality of flow directing units to map to lymphatic vessels of the user.

The garment may further include a bioimpedance sensing device configured to detect a volume of a region of a body of the user. The volume may be tracked over time. The region of the body may be an arm, leg, foot, hand, shoulder, trunk, head, and/or neck of the user. The bioimpedance sensing device may communicate with a computing device. The bioimpedance sensing device may include a sensor, microcontroller, wired and/or wireless communication interface, battery, and shell. The bioimpedance sensing device may be configured to send and receive an electrical signal across the garment through a region of a body of the user.

The garment may further include one or more vibration units configured to vibrate to promote lymphatic flow. The garment may be configured to position the one or more vibration units at or proximate lymph nodes of the user.

In one embodiment, a garment configured to be worn by a user to promote lymphatic flow includes: a plurality of flow directing units, each of the plurality of flow directing units comprising a first wall, a second wall that is offset from the first wall, and a plurality of struts extending at an angle between the first wall and the second wall, the second wall configured to face skin of the user when the garment is worn; wherein the plurality of struts are configured to translate a compressive force applied to the garment to promote fluid within the user to flow in a direction.

The plurality of struts may be in a repeating pattern. The plurality of struts may be evenly distributed. The plurality of struts may be arranged at an acute angle or an obtuse angle relative to the first wall. The garment may further include a plurality of teeth. The plurality of teeth may be disposed on the second wall. The plurality of teeth may be in line with a central axis of the plurality of struts. The plurality of flow directing units may be made of a low durometer material. The plurality of flow directing units may be made of foam, plastic, or rubber.

The garment may include channels configured to receive the plurality of flow directing units to maintain an orientation of the plurality of flow directing units. The plurality of flow directing units may be sewn into the garment. The garment may be configured to be worn on an arm, leg, foot, hand, shoulder, trunk, head, and/or neck of the user. The garment may be configured to position the plurality of flow directing units to map to lymphatic vessels of the user.

The garment may further include a bioimpedance sensing device configured to detect a volume of a region of a body of the user. The volume may be tracked over time. The region of the body may be an arm, leg, foot, hand, shoulder, trunk, head, and/or neck of the user. The bioimpedance sensing device may communicate with a computing device. The bioimpedance sensing device may include a sensor, microcontroller, wired and/or wireless communication interface, battery, and shell. The bioimpedance sensing device may be configured to send and receive an electrical signal across the garment through a region of a body of the user.

The garment may further include one or more vibration units configured to vibrate to promote lymphatic flow. The garment may be configured to position the one or more vibration units at or proximate lymph nodes of the user.

In some variants, a garment that may be worn by a user to promote lymphatic flow is disclosed herein. The garment may include a flow directing unit. The flow directing unit may include an inner wall, an outer wall offset from the inner wall, and a plurality of struts extending between and connecting to the inner wall and the outer wall. The plurality of struts may include first struts and second struts. The first struts may be oriented in a first direction and the second struts may be oriented in a second direction different than the first direction. The plurality of struts may translate a force applied to the garment to promote fluid within the user to flow in one or more directions when the garment is worn by the user.

In some variants, each of the plurality of struts may include a first portion connected to the inner wall, a second portion connected to the outer wall, and an intermediate portion connecting the first portion and the second portion. The intermediate portion may be angled relative to the inner wall.

In some variants, the first struts may be oriented perpendicularly relative to the second struts.

In some variants, the first struts may be oriented perpendicularly relative to the second struts in longitudinal directions of the first struts and the second struts.

In some variants, the second portions of the first struts and the second struts can be connected.

In some variants, the first portion of one second strut of the second struts may be disposed under the second portion of one first strut of the first struts. The second portion of the one second strut may be connected to the second portion of a second first strut of the first struts.

In some variants, the plurality of struts may be arranged at an acute angle relative to the inner wall.

In some variants, the plurality of struts may be arranged at an obtuse angle relative to the inner wall.

In some variants, the garment may include a bioimpedance sensing device that can communicate with a computing device.

In some variants, the garment may include one or more vibration units.

In some variants, the one or more directions may include toward a trunk of the user.

In some variants, the one or more directions may include radially outward.

In some variants, the plurality of struts can create a linear, spiral, double helical, random, and/or combination of any of the foregoing flows.

In some variants, a garment that may be worn by a user to promote lymphatic flow is disclosed herein. The garment can include a lattice structure. The lattice structure may translate a force applied to the garment to promote fluid within the user to flow in one or more directions when the garment is worn by the user.

In some variants, the lattice structure may include a strut-based lattice.

In some variants, the lattice structure may include a skeletal-triply periodic minimal surface (TPMS) based lattice.

In some variants, the lattice structure may include a sheet-triply periodic minimal surface (TPMS) based lattice.

In some variants, the lattice structure may include an overlapping coordinated lattice.

In some variants, the lattice structure may include a skeletal-based lattice.

In some variants, the lattice structure may include a sheet-based lattice.

In some variants, the lattice structure may be 3D printed.

In some variants, the lattice structure may include multiple layers.

In some variants, the lattice structure may include different densities at different locations.

In some variants, the lattice structure can include multiple types of lattices.

In some variants, the lattice structure may include different hardnesses at different locations.

In some variants, the lattice structure may include different thicknesses at different locations.

In some variants, the one or more directions can include a first direction and a second direction.

In some variants, the lattice structure can include an area of increased hardness that corresponds to a fibrotic area of the user.

In some variants, the garment may include a bioimpedance sensing device that can communicate with a computing device.

In some variants, the garment can include one or more vibration units.

In some variants, the one or more directions can include toward a trunk of the user.

In some variants, the one or more directions may include radially outward.

In some variants, the lattice structure may create a linear, spiral, double helical, random, and/or combination of any of the foregoing flows.

Neither the preceding summary nor the following detailed description purports to limit or define the scope of protection. The scope of protection is defined by the claims. Furthermore, reference is made herein to treating lymphedema. However, one of skill in the art will understand, after reviewing the entirety of this disclosure, that the devices (e.g., garments, flow directing units, etc.), methods, systems, components, etc. described herein may be used for other purposes besides treating lymphedema. For example, the garments described herein can be worn by athletes to promote circulation to foster recovery after physical activity. Additionally, one of ordinary skill in the art will understand, after reviewing the entirety of this disclosure, that the devices (e.g., garments, flow directing units, etc.), methods, systems, components, etc. described herein can be used by both humans and animals.

### BRIEF DESCRIPTION OF THE DRAWINGS

The abovementioned and other features of the embodiments disclosed herein are described below with reference to the drawings of the embodiments. The illustrated embodiments are intended to illustrate, but not to limit, the scope of protection. Various features of the different disclosed embodiments can be combined to form further embodiments, which are part of this disclosure.
FIG. 1A illustrates a lymphatic system of a body and an example flow directing unit, which can also be referred to as a garment insert.
FIG. 1B illustrates the lymphatic system of the body and directions of flow that can result from the use of the compression garments and inserts disclosed herein.
FIG. 1C illustrates an example micro-circulation cycle that may occur in a body.
FIG. 2A illustrates the flow directing unit in an uncompressed state.
FIG. 2B illustrates the flow directing unit in a compressed state to urge/promote lymphatic fluid to flow in a direction.
FIG. 3 illustrates a garment incorporating one or more flow directing units worn on an arm of a user.
FIG. 4 illustrates a garment incorporating one or more flow directing units worn on a leg of a user.

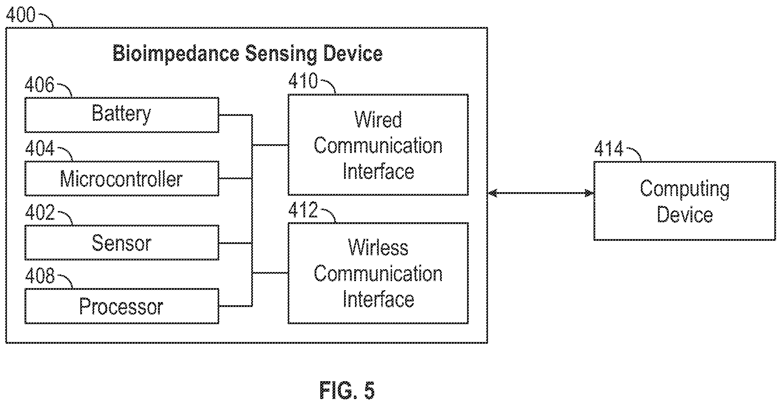

FIG. 5 illustrates a schematic for a bioimpedance sensing device.
FIGS. 6A and 6B illustrate a double wall flow directing unit with teeth.
FIGS. 7A and 7B illustrate a double wall flow directing unit without teeth.
FIGS. 8A and 8B illustrate a flow directing unit.
FIGS. 9A and 9B illustrate a flow directing unit.
FIGS. 10A and 10B illustrate a flow directing unit.
FIG. 11A illustrates a flow directing unit disposed on tissue of a user and a direction of flow of fluid within the body of the user.
FIG. 11B illustrates the flow directing unit of FIG. 11A and a direction of flow of fluid within the body of the user.
FIGS. 12A and 12B illustrate the flow directing unit of FIG. 11A.
FIG. 12C illustrates an enlarged section view of the flow directing unit of FIG. 11A.
FIG. 13A illustrates example strut-based lattices that may be incorporated into a flow directing unit.
FIG. 13B illustrates example skeletal-triply periodic minimal surface (TPMS) based lattices that may be incorporated into a flow directing unit.
FIG. 13C illustrates example sheet-triply periodic minimal surface (TPMS) based lattices that may be incorporated into a flow directing unit.
FIG. 14 illustrates an example flow directing unit that incorporates lattices of different densities.

### DETAILED DESCRIPTION

Although certain embodiments and examples are described below, this disclosure extends beyond the specifically disclosed embodiments and/or uses and obvious modifications and equivalents thereof. Thus, it is intended that the scope of this disclosure should not be limited by any particular embodiments described below. Furthermore, this disclosure describes many embodiments in reference to treating lymphedema, but as described herein, any embodiment and modifications or equivalents thereof should not be limited to treating lymphedema.

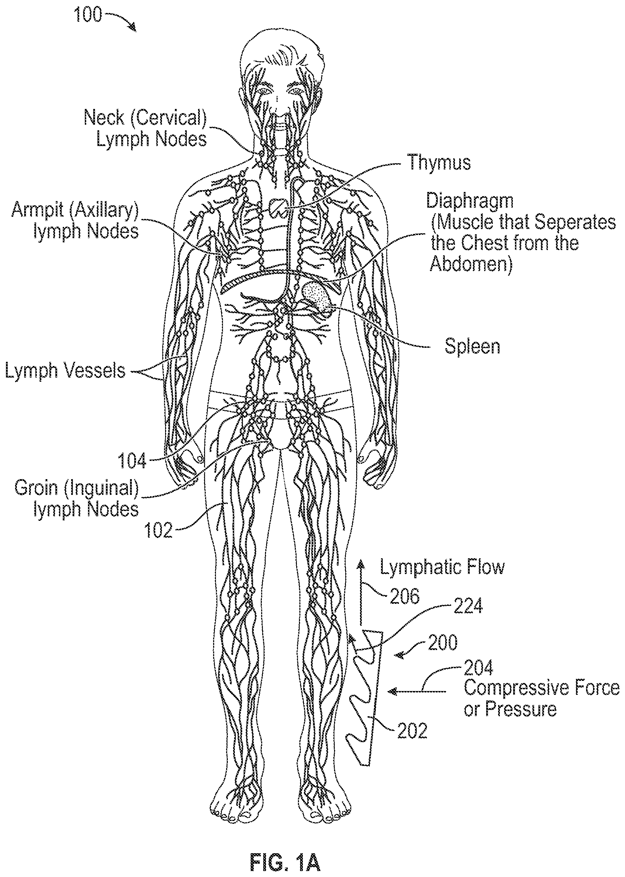

FIG. 1A illustrates a lymphatic system of a body 100 of a user. The lymphatic system is an organ system, which may be a separate and complete body system with its own functional contributions to the human body 100. The lymphatic system includes a network of lymphatic vessels or channels 102 and nodes 104, among other features, that help to recirculate blood and/or lymph through the human body. Lymph capillaries may anastomose with pre-collectors, collector vessels, which travel to lymph nodes 104, exit the nodes 104, converge with trunks which eventually merge into the largest lymph vessel-the thoracic duct. Lymphatics may originate from the mucous membranes, the soft connective tissue spaces, and dermis of the skin. Lymphatics may be positioned in close proximity to the blood capillary bed. As described herein, lymphedema is a medical condition in which excess fluid, such as lymph fluid, collects in an area of the body, such as a limb, and causes swelling (i.e., edema). Left untreated, numerous additional detrimental symptoms may develop.

FIG. 1A illustrates a flow directing unit 200, which can also be referred to as a garment insert and/or vector foam. The flow directing unit 200 can be incorporated into a garment, such as a sleeve, wrap, pant, shirt. hat, glove, sock, etc., that can be worn by a user to promote/urge movement (e.g., drainage, flow, etc.) of fluid, such as lymph or blood, from an area of the body to treat lymphedema and/or other conditions.

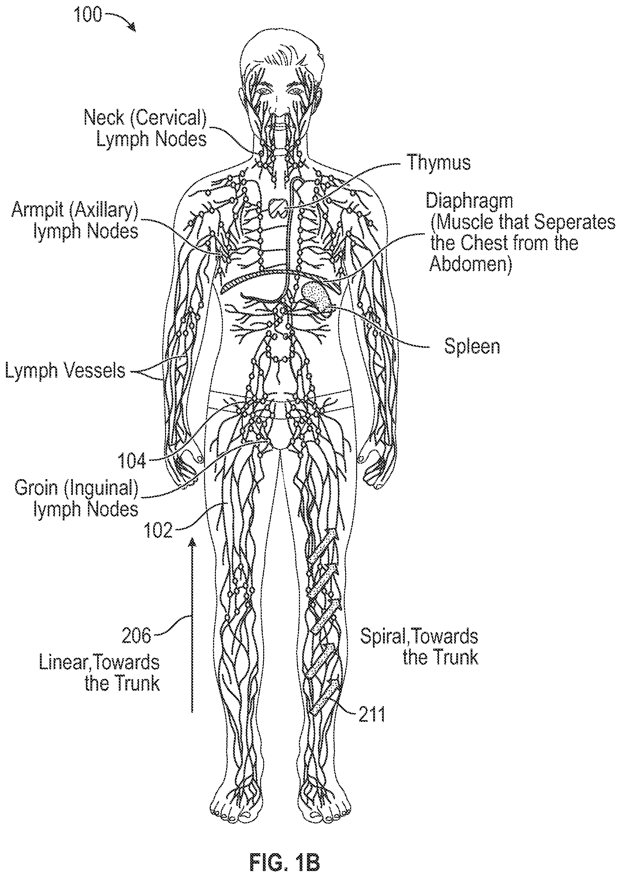

The flow directing unit 200 can include one or more teeth 202, which can at least also be referred to as protrusions, angled protrusions, ridges, or projections. The garment incorporating the flow directing unit 200 can orient the one or more teeth 202 toward the skin of the user when worn. The one or more teeth 202, when compressed, can provide a directional force to urge the flow of fluid within the user in a direction. The one or more teeth 202 can be angled such that, when compressed, the one or more teeth 202 translate compressive forces in a direction. For example, a compressive force or pressure can be applied to the flow directing unit 200 in the direction of arrow 204, compressing the flow directing unit 200. The angle of the one or more teeth 202 can translate at least a portion of the compressive force in the direction of arrow 224 (e.g., a portion of the compressive force can be applied in the direction of arrow 204 and a portion of the compressive force can be applied in the direction of arrow 206) to the skin of the user through the one or more teeth 202, urging fluid in the body 100 of the user, such as lymph or blood, to flow in the direction of arrow 206 toward a trunk of the user. In some variants, the garment incorporating the one or more flow directing units 200 can, when worn, orient the one or more teeth 202 such that compressive forces applied in the direction of arrow 204 are translated in a direction to direct flow away from the trunk of the user in a direction that is opposite arrow 206. In some variants, the garment, when worn, can orient the one or more flow directing units 200 to urge the flow of fluid within the body 100 of the user in any direction. The flow directing unit 200 can be varying lengths, widths, etc. The flow directing unit 200 can be made of a variety of materials, which can include foam, plastic, rubber, and/or other low durometer materials. The one or more teeth 202 may be oriented in different orientations to urge fluid (e.g., lymphatic fluid, interstitial fluid) to flow in any direction within the body of the user. For example, as illustrated in FIG. 1B, the flow directing unit 200 may urge the flow of fluid in the direction of arrow 206 (e.g., linear towards the trunk of the user). The flow directing unit 200 may urge the flow of fluid in the direction of arrow 211 (e.g., spiral towards the trunk of the user). In some variants, fluid within the body of the user (e.g., lymphatic, interstitial fluid) may be urged away from the trunk of the user. The flow directing unit 200 may create different flows, which may at least include linear, spiral, double helical, random, others, and/or a combination of any of the forgoing to guide fluid (e.g., lymphatic fluid, interstitial) within the body of the user (e.g., toward the trunk).

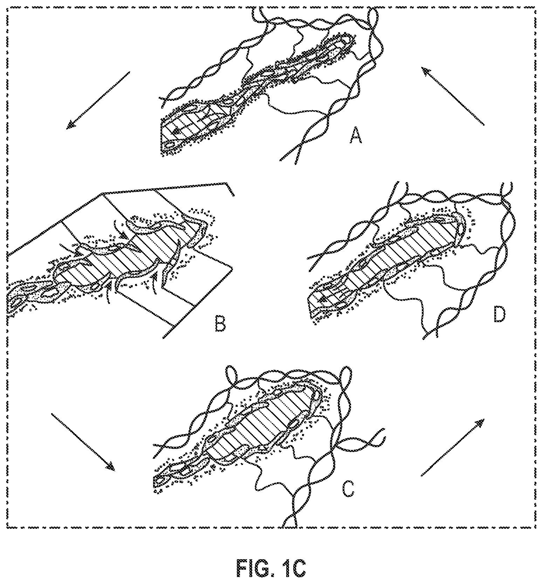

FIG. 1C illustrates an example micro-circulation cycle that may occur in a body. In normal micro-circulation, an increase in interstitial fluid pressure (or an increase of tissue fluid) may pull against the filaments, creating tension. The tension may be transferred to the overlapping basement membrane of the lymph capillary to open the swinging flaps. At the same time, because there may be a negative (less pressure resistance) within the lymph capillary, there may be a negative gradient or suction effect. The interstitial fluid pressure may push in, causing an influx of fluid, which may be absorbed into the lymph capillary network. The directional fluid flow (absorption) may stop when the lymph capillary has reached a filled capacity because the pressure inside the vessel (lymph load) is equal or greater than the surrounding tissue. As the lymph progressively drains deeper into the lymphatic vessel network, the negative pressure in the lymph capillary may reoccur and the cycle of reabsorption is re-initiated

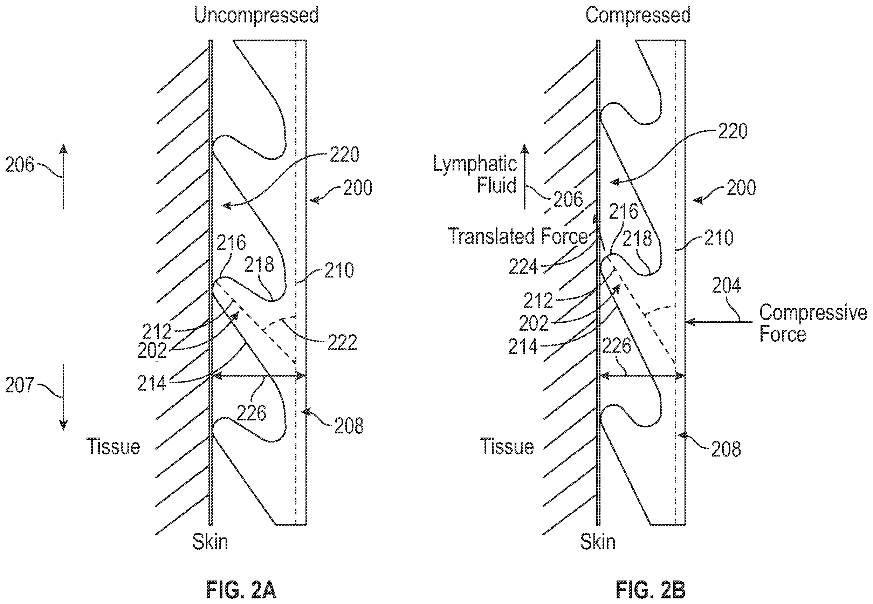

FIG. 2A illustrates a flow directing unit 200 in an uncompressed state proximate the skin of the user. The flow directing unit 200 can include a wall 208, which can also be referred to as a base or elongate wall. As described herein, the flow directing unit 200 can include one or more teeth 202. The one or more teeth 202 can be distributed along the wall 208 with a gap 220 between adjacent teeth 202. The one or more teeth 202 can be equidistantly spaced from each other along the wall 208. In some variants, the one or more teeth 202 can be unequally spaced from each other. The one or more teeth 202 can be in a repeating pattern (e.g., repeating saw toothed pattern). The one or more teeth 202 can be disposed between the wall 208 of the flow directing unit 200 and the skin of the user. The one or more teeth 202 can include an inclined surface 214. The inclined surface 214 can be flat. The one or more teeth 202 can taper in thickness to ends 216. In some variants, the one or more teeth 202 maintain a consistent thickness to ends 216.

The one or more teeth 202 can extend from the wall 208 at an angle. For example, a longitudinal axis 210 may extend through the wall 208 and a center axis 212 may extend through each of the one or more teeth 202. The center axis 212 and/or the inclined surface 214 may be oriented at an angle 222 relative to the longitudinal axis 210. The angle 222 can be acute (i.e., between zero and ninety degrees) to urge the flow of fluid in the direction of arrow 206 when a compressive force is applied to the wall 208. The flow directing unit 200 can be flipped to urge the flow of fluid in the direction of arrow 207 when a compressive force is applied to the wall 208. In some variants, the one or more teeth 202 can extend from the wall 208 at an obtuse angle (i.e., between ninety and one hundred and eighty degrees) to urge the flow of fluid in the direction of arrow 207 when a compressive force is applied to the wall 208. The one or more teeth 202 of the flow directing unit 200 may help to stimulate the lymph capillary to open the swinging flaps, described in reference to FIG. 1C, to push (e.g., directly push) fluid up a limb of the user towards the user's trunk through the use of external compressive force (e.g., the weight of the user when lying down on the flow directly unit 200).

The flow directing unit 200 can have a width 226 in an uncompressed state. The width 226 can be the lateral distance between the outside surface of the wall 208 and the innermost surface of an end 216 of the one or more teeth 202. The ends 216 of the one or more teeth 202 can be rounded, which can improve user comfort. The one or more teeth 202 can include a curve 218, which can be disposed opposite the inclined surface 214. The curve 218 can help to translate the compressive force applied to the flow directing unit 200 in the direction of arrow 206 or arrow 207 by resisting the buckling or excessive buckling of the one or more teeth 202 under a compressive load.

FIG. 2B illustrates a flow directing unit 200 under compression. As illustrated, a compressive force can be applied to the wall 208 in the direction of arrow 204, compressing the one or more teeth 202 between the wall 208 and the skin of the patient. As described herein, the garment incorporating the one or more flow directing units 200 can be worn by a user while sleeping or otherwise laying down. Accordingly, the compressive force applied to the wall 208 can result from the weight of the user pressing against a surface upon which the user is sleeping (e.g., a bed). In some variants, the garment incorporating the one or more flow directing units 200 can be tightened around the user to apply a compressive force. The garment can be tightened using a variety of techniques using straps, laces, buckles, motors, shortening of shape memory materials, pulleys, and/or others.

The flow directing unit(s) 200 by way of the one or more teeth 202 can provide a directional force to urge the flow of fluid in a direction within the user when compressed. The angle 222 of the one or more teeth 202 can cause the one or more teeth 202 to translate at least a portion of the compressive force in a direction that is different than that of arrow 204 when compressed against the skin of the user. For example, when the angle 222 is acute, the translated force can be directed in the direction of arrow 224 against the skin of the user to urge the flow of fluid within the user in the direction of arrow 206. In some variants, when the angle 222 is obtuse or the flow directing unit 200 is reoriented, the translated force can be directed against the skin of the user to urge the flow of fluid within the user in the direction of arrow 207. However, even with a portion of the translated force directed in the direction of arrow 224 or otherwise, a portion of the compressive force may be applied to the skin of the user in the direction of arrow 204. Under compression, the angle 222 of the one or more teeth 202 may be decreased but, upon removal of the compressive force, the angle 222 may return to an uncompressed size. The compressive force can decrease the width 226 of the flow directing unit 200.

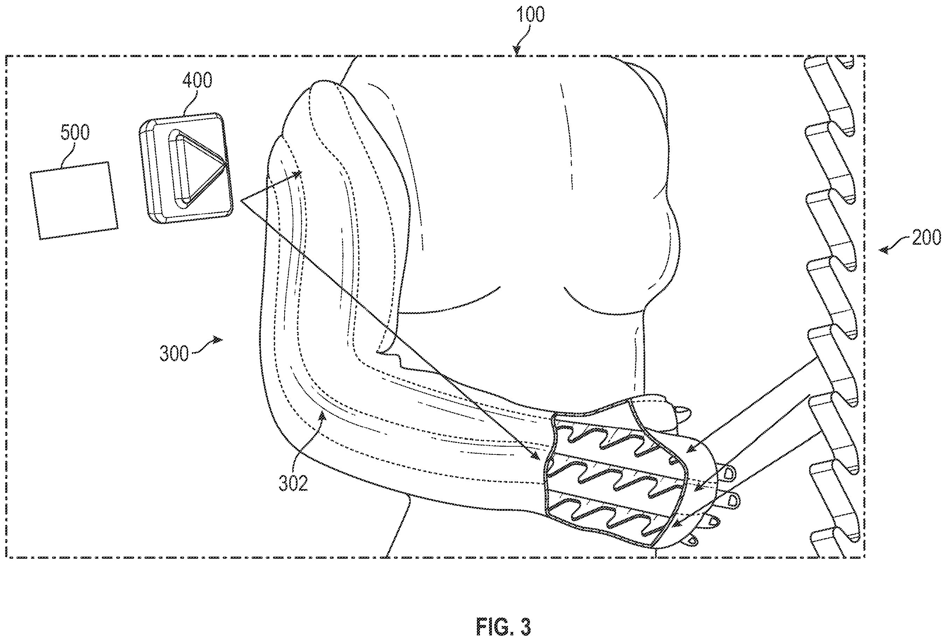

FIG. 3 illustrates a garment 300 worn on the arm of the user. The garment 300 can also be described as a sleeve, arm sleeve, wrap, glove, etc. The garment 300 can surround the limb and/or only a portion thereof. The garment 300 can have one or more flow directing units 200. In some variants, the one or more flow directing units 200 can be sewn into the garment 300. The flow directing units 200 can be incorporated into the garment 300 such that the one or more teeth 202 are facing the skin of the user. In some variants, the one or more teeth 202 directly contact the skin and/or clothes of the user. In some variants, the garment 300 includes a layer between the one or more teeth 202 of the user and the skin and/or clothes of the user. In some variants, a layer of the garment 300 covers the one or more flow directing units 200.

The one or more flow directing units 200 can be positioned within channels 302 of the garment 300. In some variants, the channels 302 can receive the one or more flow directing units 200 therein and maintain an orientation of the one or more flow directing units 200 while the garment 300 is worn. To direct fluid out of the arm and into the trunk of the user under compression, the one or more flow directing units 200 can be oriented with a longitudinal direction thereof oriented in the longitudinal direction of the user's arm. To direct fluid out of the arm and into the trunk of the user under compression, the one or more teeth 202 of the one or more flow directing units 200 can extend at an angle toward the trunk of the user. The channels 302 and/or flow directing units 200 can be positioned to map or track to lymphatic vessels (e.g., channels, routing, etc.) and/or nodes and/or proximate nodes of the lymphatic system to move fluid therein.

The garment 300 can include one or more bioimpedance sensing devices 400. The bioimpedance sensing device(s) 400 can detect a volume of the arm of the user. When the garment 300 is worn on another portion of the body (e.g., leg, foot, trunk, head, neck, shoulder, etc.), the bioimpedance sensing device(s) 400 can detect a volume of that portion of the body. The bioimpedance sensing device(s) 400 can be distributed throughout the garment 300 to detect volume at various positions. The bioimpedance sensing devices 400 can send and receive an electrical signal across the garment 300 through portions of the patient's body to detect volume. The bioimpedance sensing device 400 can track volume over time.

The garment 300 can include one or more vibration units 500 (e.g., vibration motors). The vibration unit(s) 500 can be positioned along the lymphatic vessels (e.g., channels, routing, etc.) and/or nodes and/or proximate nodes of the lymphatic system to promote or create lymphatic flow. The vibration unit(s) 500 can vibrate, which can include vibrating at different frequencies, patterns, durations, etc.

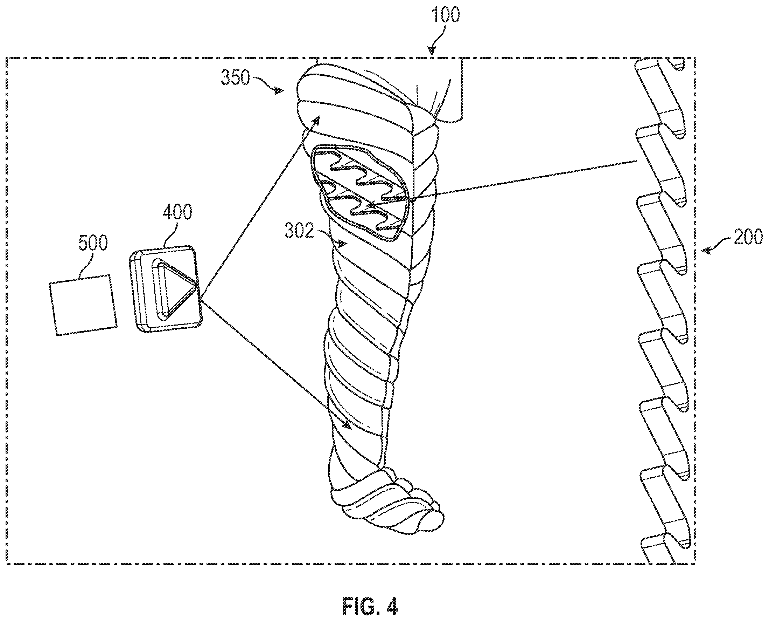

FIG. 4 illustrates a garment 300 worn on the leg and/or foot of the user. The garment 300 can also be described as a sleeve, leg sleeve, wrap, sock, etc. The garment 300 can include one or more flow directing units 200, which can be oriented as described herein. In some variants, the one or more flow directing units 200 can be sewn into the garment 300. In some variants, the one or more flow directing units 200 can be disposed in channels 302 of the garment 300. The channel 302 can maintain the orientation of the one or more flow directing units 200 while the garment 300 is worn. The channels 302 can be oriented to wrap around the leg and/or foot of the user, which can include being wrapped around the leg and/or foot of the user at an angle relative to the longitudinal direction of the leg and/or foot. As described herein, the channels 302 and/or flow directing units 200 can be positioned to map or track to lymphatic vessels (e.g., channels, routing, etc.) and/or nodes and/or proximate nodes of the lymphatic system to move fluid therein. The garment 300 can include one or more bioimpedance sensing devices 400, as described herein. The garment 300 can include one or more vibration units 500, as described herein.

FIG. 4 illustrates a schematic of the bioimpedance sensing device 400. The bioimpedance sensing device 400 can detect the volume of a portion of the user's body (e.g., limb). The bioimpedance sensing device 400 can detect volume over time. The bioimpedance sensing device 400 can send and receive an electrical signal across the garment 300 through the patient's body (e.g., limb) or area of coverage. The bioimpedance sensing device 400 can include a battery 406 which can include rechargeable batteries or single use batteries. In some variants, the battery 406 can charge from movement of the user. The battery 406 can be external or internal to the bioimpedance sensing device 400. The bioimpedance sensing device 400 can include a microcontroller 404. The bioimpedance sensing device 400 can include a sensor 402. In some variants, the electrical signal can be sent and/or received from the sensor 402. The bioimpedance sensing device 400 can include a processor 408. The bioimpedance sensing device 400 can include a wired communication interface 410 and/or wireless communication interface 412 (e.g., WI-FI, Bluetooth, cellular networks such as 3G, 4G, and 5G, etc.) which can facilitate communication with a computing device 414, such as a computer, tablet, smart phone, remote computing environment, etc. The bioimpedance sensing device 400 can send data to the computing device 414, which can include usage, volume, device status, and/or other data. The computing device 414 can be accessed by an individual involved in the care of the user for monitoring, diagnosis, treatment, etc.

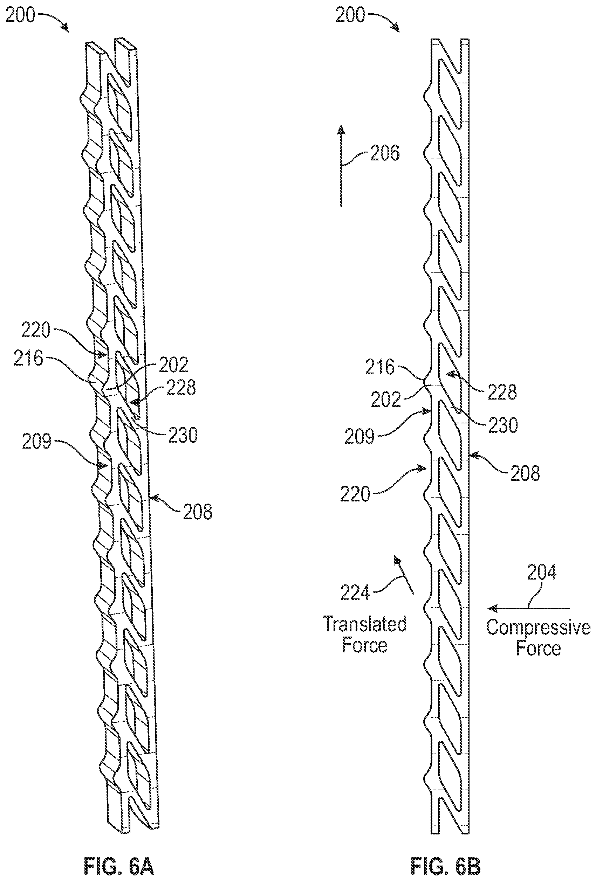

FIGS. 6A-10B illustrate variations of the flow directing unit 200. The features of the various flow directing units 200 described herein can be combined, removed, etc. As described herein, the flow directing units 200 can be various sizes and incorporate various materials. The flow directing unit 200, including different variations, can be incorporated into a garment 300 to be worn by a user.

FIGS. 6A and 6B illustrate a double wall flow directing unit 200 with teeth 202. The flow directing unit 200 can include a wall 208. The flow directing unit 200 can include a second wall 209 (e.g., inner wall proximate the user's skin). The second wall 209 can be offset from the wall 208. The second wall 209 can be parallel to the wall 208. A plurality of struts 230, which can also be referred to as supports, braces, or cross supports, can extend between the wall 208 and the second wall 209. The struts 230 can be angled relative to the wall 208 and second wall 209 such that, when a compressive force is applied to the wall 208 in the direction of arrow 204, the struts 230 translate at least a portion of the compressive force in the direction of arrow 224 to urge the flow of fluid in the direction of arrow 206. In some variants, the struts 230 can extend from the wall 208 to the second wall 209 at an acute angle relative to the wall 208 such that the flow directing unit 200 translates compressive force in the direction of arrow 224 to urge the flow of fluid in the direction of arrow 206. In some variants, the second wall 209 can move in the direction of arrow 206 when the flow directing unit 200 is under compression.

In some variants, the flow directing unit 200 can be flipped to redirect the translation of compressive forces to urge the flow of fluid in the direction opposite of arrow 206. In some variants, the struts 230 can extend from the wall 208 to the second wall 209 at an obtuse angle relative to the wall 208 such that the flow directing unit 200 translates compressive force in a direction to urge the flow of fluid in the direction opposite of arrow 206.

The flow directing unit 200 can include opening 228, e.g., gaps, between adjacent struts 230, which can enable the second wall 209 to translate (e.g., move in the direction of arrow 206 or opposite) and/or enable the distance between the wall 208 and second wall 209 to decrease when the flow directing unit 200 is under compression.

The flow directing unit 200 can include teeth 202. The teeth 202 can be positioned on the second wall 209 to face the skin of the user. The teeth 202 can be disposed on a side of the second wall 209 that is opposite the struts 230. The teeth 202 can be positioned as a continuation of the struts 230. The teeth 202 can be positioned in line with a central axis of the struts 230. The teeth 202 can be symmetric about a midplane. The angled struts 230 can provide the force translation in a direction other than arrow 204 (e.g., a portion of the force in the direction of arrow 206 or opposite arrow 206) enabling the teeth 202 to be symmetric about a midplane. In some variants, the teeth 202 can be angled. The ends 216 of the teeth 202 can be rounded. The teeth 202 can help to translate the compressive force.

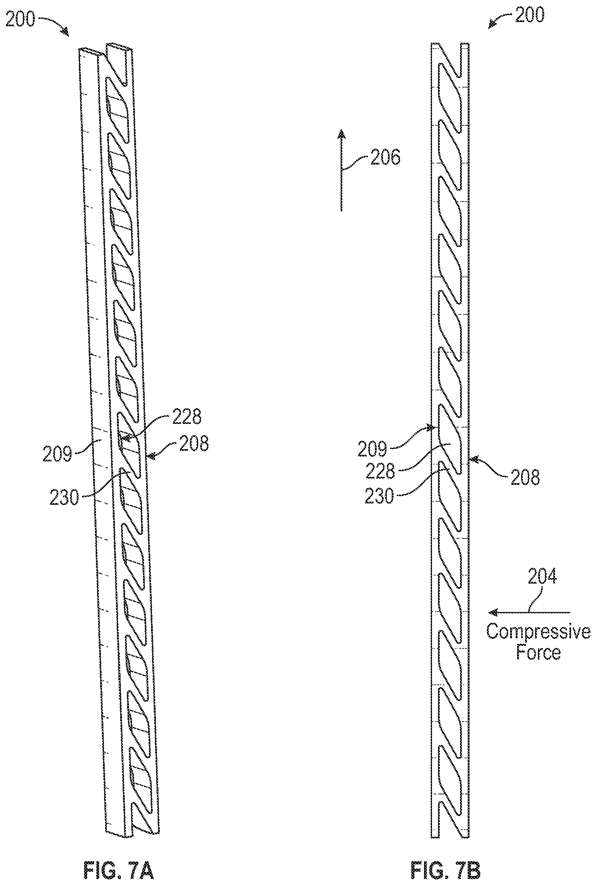

FIGS. 7A and 7B illustrates a double wall flow directing unit 200 without teeth 202. The double wall flow directing unit 200 illustrated in FIGS. 6A and 6B can be the same as the double wall flow directing unit 200 illustrated in FIGS. 7A and 7B except for the omission of the teeth 202.

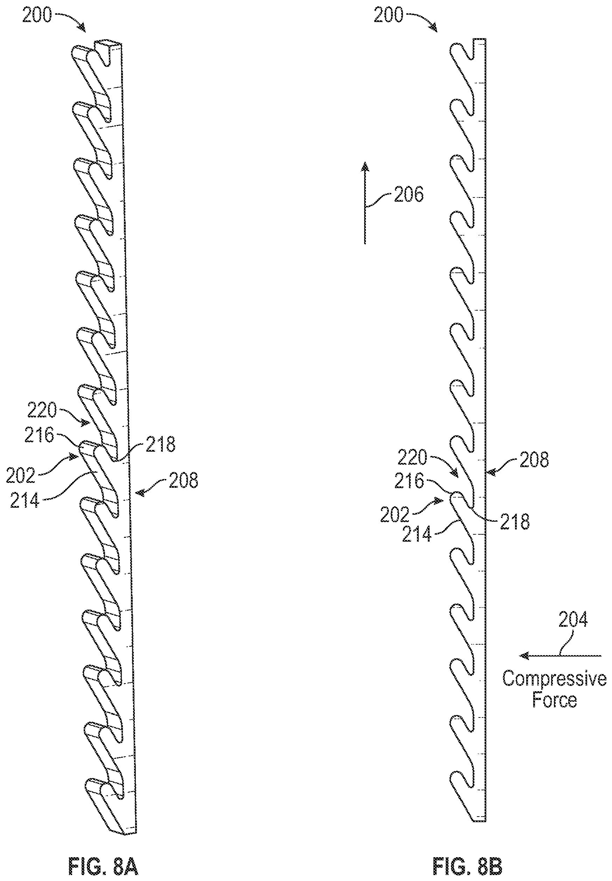

FIGS.8A and 8B illustrate a flow directing unit 200 that is similar to the flow directing unit 200 described in reference to FIGS. 1, 2A, and 2B. The flow directing unit 200 illustrated in FIGS. 8A and 8B, however, can have teeth 202 with a consistent thickness and/or a larger gap 220 between adjacent teeth 202 compared to the flow directing unit 200 described in reference to FIGS. 1, 2A, and 2B.

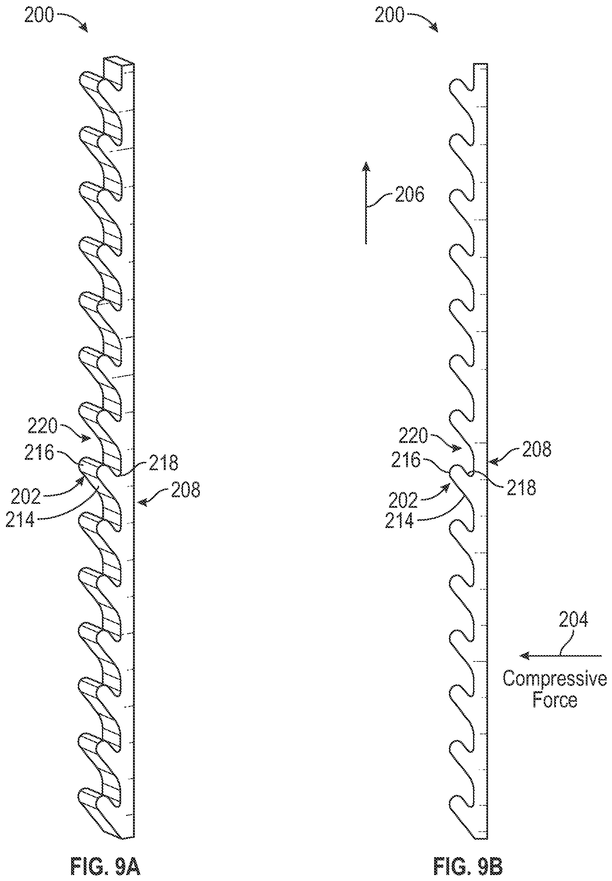

FIGS. 9A and 9B illustrate a flow directing unit 200 that is similar to the flow directing unit 200 described in reference to FIGS. 8A and 8B. The flow directing unit 200 illustrated in FIGS. 9A and 9B, however, can have thicker teeth 202, a larger gap 220 between adjacent teeth 202, and a thicker wall 208 compared to the flow directing unit 200 described in reference to FIGS. 8A and 8B.

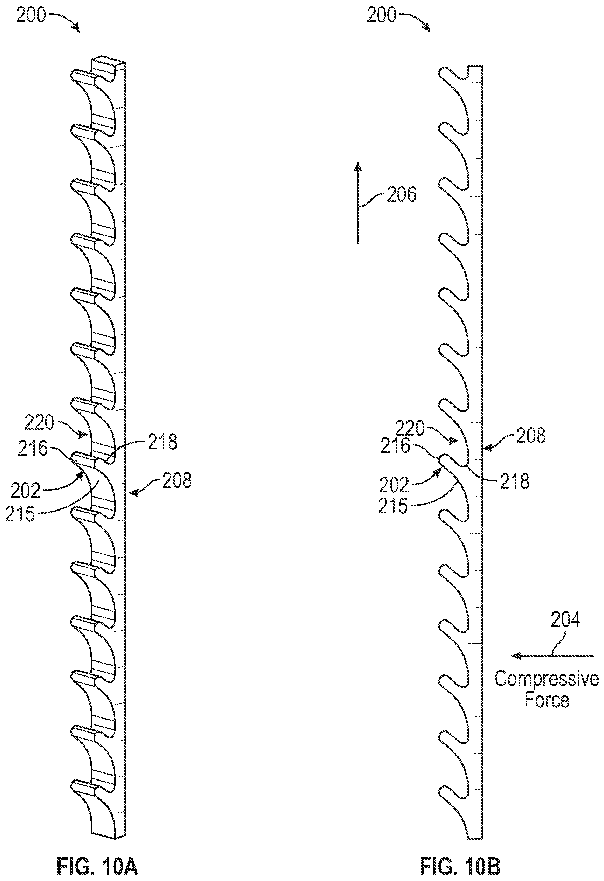

FIGS. 10A and 10B illustrate a flow directing unit 200 that is similar to the flow directing unit 200 described in reference to FIGS. 8A and 8B. The flow directing unit 200 illustrated in FIGS. 10A and 10B, however, can have thinner teeth 202 and a larger angle (e.g., less acute, more obtuse) between the wall 208 and teeth 202 compared to the flow directing unit 200 described in reference to FIGS. 8A and 8B. The flow directing unit 200 illustrated in FIGS. 10A and 10B can have a curved surface 215 instead of a flat surface. In some variants, the surface 215 can be flat.

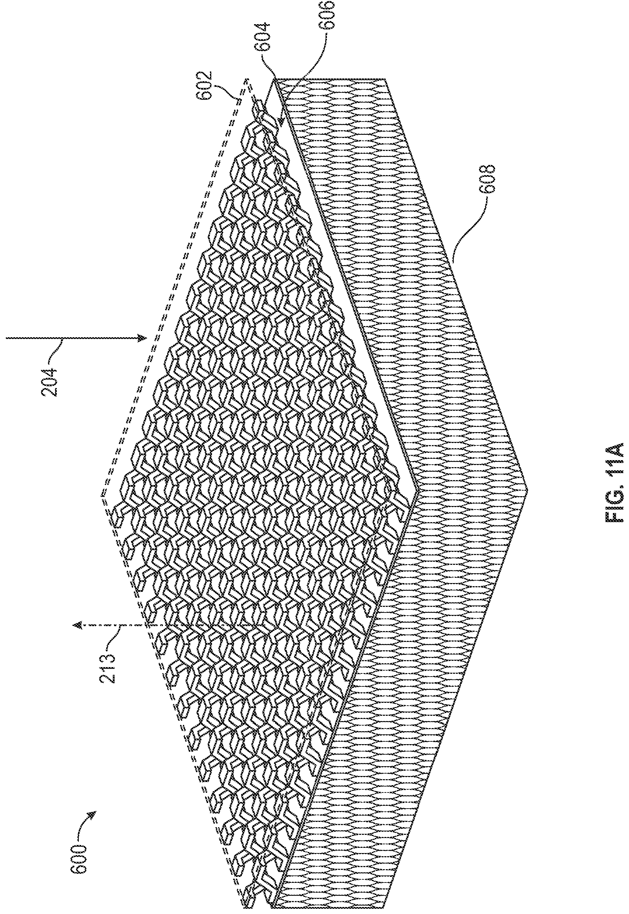

FIG. 11A illustrates a flow directing unit 600 disposed on tissue 608 of a user to direct a flow of fluid within the tissue 608. The flow directing unit 600 may be incorporated into a garment (e.g., compressive garment) that can be worn by a user, as described herein. In some variants, the garment incorporating one or more flow directing units 600 can be tightened around the user to apply a compressive force. The garment can be tightened using a variety of techniques using straps, laces, buckles, motors, shortening of shape memory materials, pulleys, and/or others. The flow directing unit 600 may utilize compressive force applied thereto to urge fluid within a user's body to flow in one or more directions.

The flow directing unit 600 may be a lattice structure. The flow directing unit 600 may include a structure 606, which may be a lattice of flow-directing units, lattice structure, and/or unit cell structures, to promote fluid flow within the tissue 608 when a force (e.g., compressive force) is applied to the flow directing unit 600 in the direction of arrow 204 (e.g., toward the tissue 608). The structure 608 may be asymmetrical. The structure 608 may not be uniform. The structure 608 may help to stimulate the lymph capillary to open the swinging flaps, described in reference to FIG. 1C, to push (e.g., directly push) fluid up a limb of the user towards the user's trunk through the use of external compressive force (e.g., the weight of the user when lying down on the flow directly unit 600). In some variants, the structure 608 may be symmetrical. In some variants, the structure 608 may be uniform.

The structure 608 may be disposed on an inner wall 604 (e.g., base). The structure 608 may be disposed between the inner wall 604 and an outer wall 602 (e.g., cover). The inner wall 604 can be disposed between the structure 606 and the tissue 608 when the flow directing unit 600 is in use. The inner wall 604 may flexible such that the inner wall 604 follow the contours of features of a user's body (e.g., arm, legs, etc.). The outer wall 602 may be flexible. In some variants, the inner wall 604 is more rigid than the outer wall 602. In some variants, the outer wall 602 is more rigid than the inner wall 604. The outer wall 602 may be offset from the inner wall 604, which may include being parallel relative to each other.

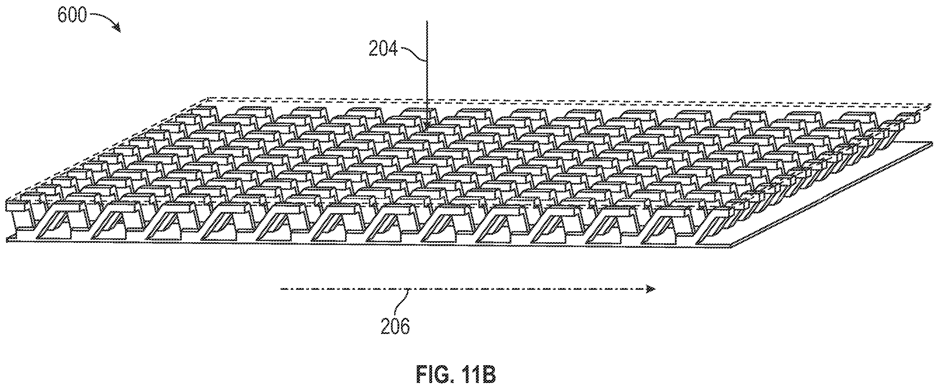

A force (e.g., compressive force) may be applied to the flow directing unit 600 in the direction of arrow 204. As described herein, the force may result from the weight of the user's body compressing the flow directing unit 600 between tissue 608 and a supporting surface (e.g., supporting surface, bed, ground surface, etc.). The force may be applied to the flow directing unit 600 at different angles, which may include perpendicular to the inner wall 604 and/or outer wall 602. The application of the force may result in the structure 604 of the flow directing unit 600 urging fluid (e.g., lymphatic fluid, interstitial fluid) to flow in the direction of arrow 213, which may include opposite the direction of arrow 204, perpendicular to the inner wall 604 and/or outer wall 602, and/or radially outward. The application of the force may result in the structure 606 of the flow directing unit 600 urging fluid (e.g., lymphatic fluid, interstitial fluid) to flow in the direction of arrow 206, as illustrated in FIG. 11B, which may be toward the trunk of the user.

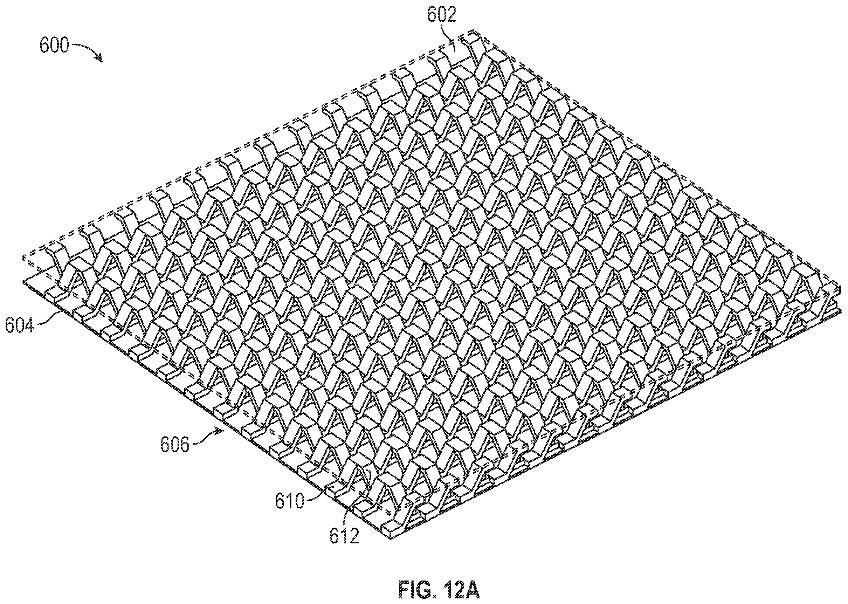

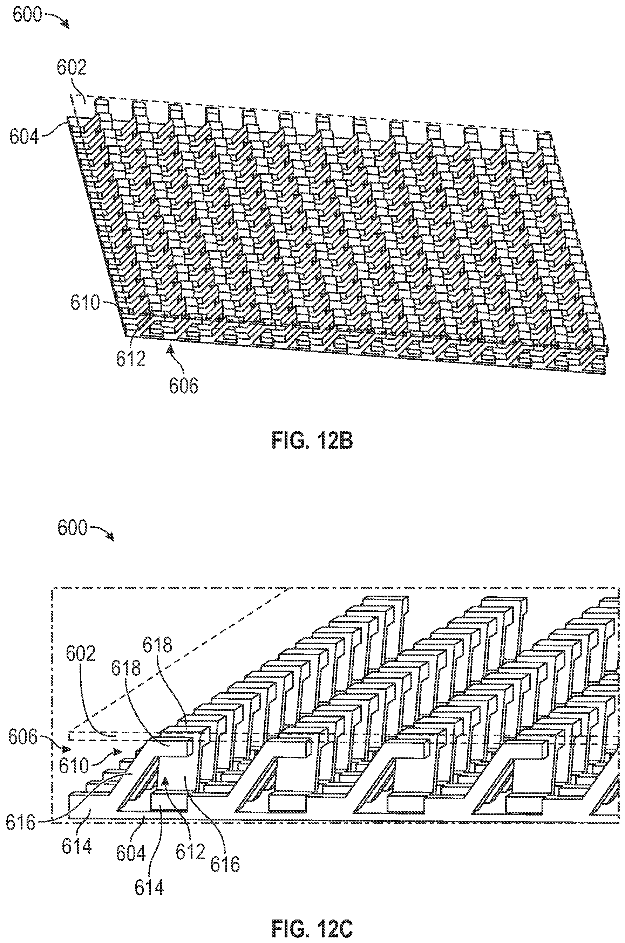

As shown in FIGS. 12A and 12B, the structure 606 may include a plurality of struts 610 (e.g., first struts), which may be protrusions, angled protrusions, and/or projections. The struts 610 may be oriented in the same first direction, which may include parallel to each other.

The structure 606 may include a plurality of struts 612 (e.g., second struts), which may be protrusions, angled protrusions, and/or projections. The plurality of struts 612 may be oriented in the same second direction, which may include parallel to each other. The second direction of the struts 612 may be different than the first direction of the struts 610. The plurality of struts 612 may be oriented perpendicular relative to the struts 610. The struts 610 and/or struts 612 may form a grid that promotes fluid flow when compressed.

As illustrated in FIG. 12C, the struts 610 (e.g., each of the struts 610) may include a first portion 614, which may be a base portion. The first portion 614 may be connected to the inner wall 604. The base portion 614 can extend outward from the inner wall 604. The struts 610 may include a second portion 618. The second portion 618 may be connected to the outer wall 602.

The struts 610 may include an intermediate portion 616 between the first portion 614 and the second portion 618. The intermediate portion 616 may connect the first portion 614 and the second portion 618. The intermediate portion 616 may be an elongate strut, brace, support, arm, tooth, and/or other structure. The cross-section of the intermediate portion may be different shapes, which may at least include circular, oval, polygonal (e.g., square, rectangular, etc.), irregular, and/or others. The intermediate portion 616 may be angled relative to the first portion 614, inner wall 604, outer wall 602, and/or second portion 618. The intermediate portion 616 may be angled, in a longitudinal direction, relative to the first portion 614, inner wall 604, outer wall 602, and/or second portion 618. For example, the longitudinal direction of the intermediate portion 616 may be angled at an acute angle (i.e., between zero and ninety degrees) relative to the inner wall 604. In some variants, the longitudinal direction of the intermediate portion 616 may be angled at an obtuse angle (i.e., between ninety and one hundred and eight degrees) relative to the inner wall 604).

The struts 612 may include similar features as the struts 610, which may include the first portion 614, intermediate portion 616, and/or second portion 618. The struts 612 may be oriented perpendicular to the struts 610. The first portion 614 of a strut 612 may be disposed, at least partially, under the second portion 618 of a first strut 610 and on the inner wall 604. The second portion 618 of the same strut 612 may connect with the second portion 618 of a second strut 610 that is parallel to the first strut 610 and with the outer wall 602.

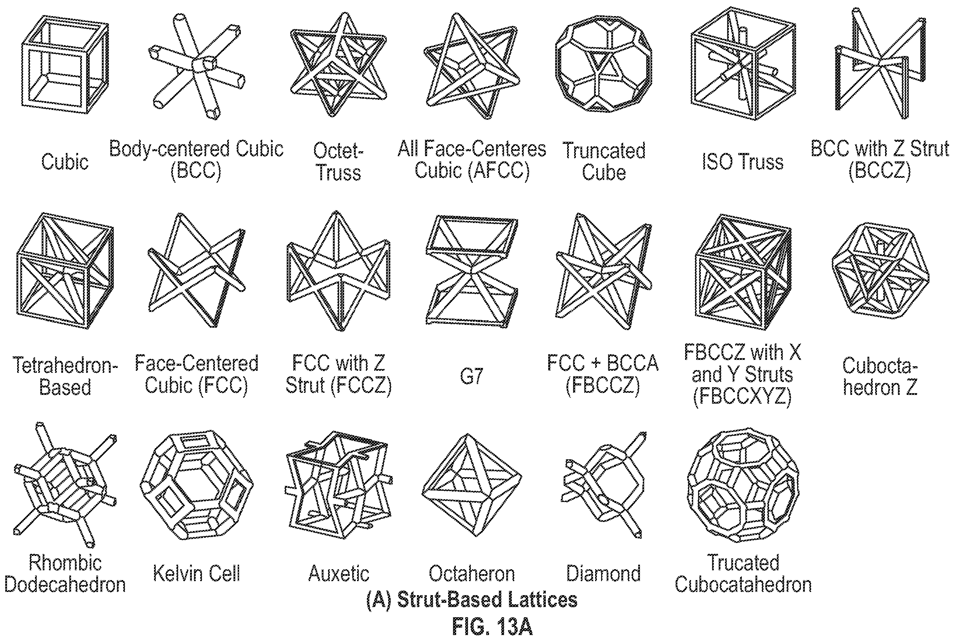

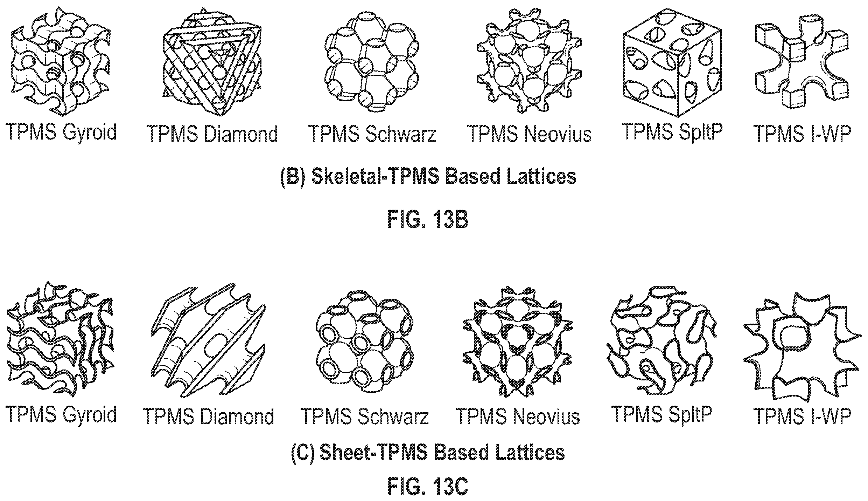

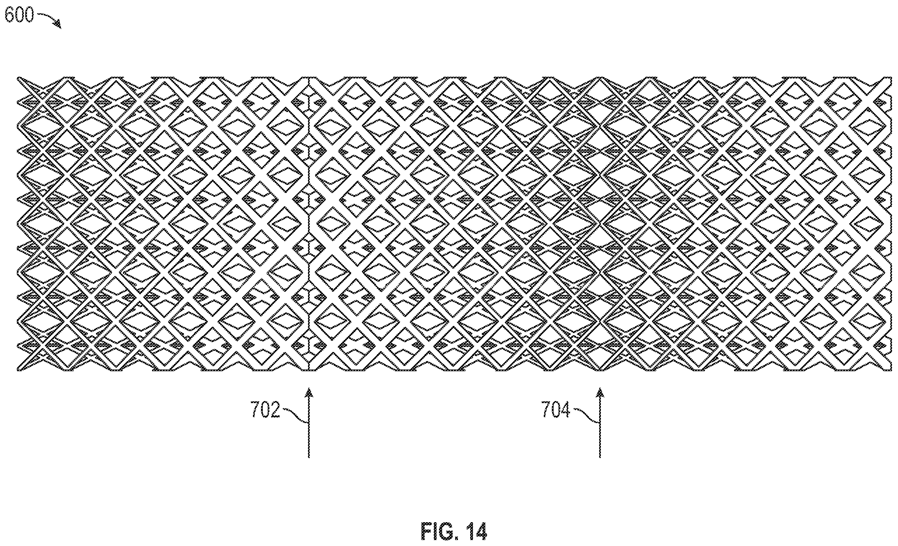

The structure 606 may include a lattice. The lattice may include several layers to create movement of fluid in more than one direction, which may depend on the direction the force (e.g., compressive force) is applied to the flow directing unit 600 (e.g., the lattice may direct flow in a first direction when compressive for is applied in one direction but may direct flow in a second direction when compressive force is applied in another direction). FIG. 13A illustrates example strut-based lattices for the structure 606. FIG. 13B illustrates example skeletal-triply periodic minimal surface (TPMS) based lattices for the structure 606. FIG. 13C illustrates example sheet-triply periodic minimal surface (TPMS) based lattices for the structure 606. The structure 606 may include strut-based lattice(s), overlapping coordinated lattice(s), skeletal-based lattice(s), and/or sheet-based lattice(s), which may include symmetric geometry and non-symmetric strut thickness. The structure 606 and/or the entirety of the flow directing unit 600 may be 3D printed. The structure 606 may be customized to the needs of a patient, which may include customizing the size and/or features of the structure 606. For example, the lattices may include varying thicknesses (e.g., cell thicknesses), densities, and/or types to create different hardnesses within the same structure 606. The use of lattices can be beneficial at least because lattices may be light weight, breathable (e.g., airflow through the lattice), variable in hardness (e.g., different hardness in the same structure 606), provide custom directional flow based on the specific needs of the patient, and/or can be custom fit to individuals. For example, FIG. 14 illustrates a structure 600 that may include different densities at different locations. The structure may include an area 702 of higher lattice density compared to an area 704 of lower lattice density. The area 702 may be harder than the area 704, which can enable specific areas of a patient to be targeted.

The hardness within the same structure 606 can be different in different locations. For example, the lattice structure within the same structure 606 may be different (e.g., body-centered cubic to tetrahedron-based) in different locations. The size of the lattice structure may be different in different locations. The density of the lattice structure may be different tin different locations. The thickness of the lattice structure may be different at different locations. The lattice structure may be altered (e.g., control the mechanical properties of a lattice by increasing the diameter of the struts in specific areas). Changing the hardness within the same structure 606 can allow the flow directing units 600 to be customized to treat a specific patient. For example, a patient may have areas of high sensitivity or pain. Accordingly, to reduce the hardness at those areas of high sensitivity, a softer lattice structure or foam (e.g., more flexible) may be used. In fibrotic areas, the hardness can be increased by using a stronger lattice structure or foam (e.g., more rigid).

The flow directing unit 600 may create different flows, which may at least include linear, spiral, double helical, random, others, and/or a combination of any of the forgoing to guide fluid (e.g., lymphatic fluid, interstitial) within the body of the user (e.g., toward the trunk).

### Terminology

Although the systems and methods have been disclosed in the context of certain embodiments and examples, it will be understood by those skilled in the art that the systems and methods extend beyond the specifically disclosed embodiments to other alternative embodiments and/or uses of the embodiments and certain modifications and equivalents thereof. Various features and aspects of the disclosed embodiments can be combined with or substituted for one another in order to form varying modes. The scope of this disclosure should not be limited by the particular disclosed embodiments described herein.

Methods of using the foregoing system(s) (including device(s), apparatus(es), assembly(ies), structure(s) or the like) are included; the methods of use can include using or assembling any one or more of the features disclosed herein to achieve functions and/or features of the system(s) as discussed in this disclosure. Methods of manufacturing the foregoing system(s) are included; the methods of manufacture can include providing, making, connecting, assembling, and/or installing any one or more of the features of the system(s) disclosed herein to achieve functions and/or features of the system(s) as discussed in this disclosure.

Certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as any subcombination or variation of any subcombination.

Moreover, while operations may be depicted in the drawings or described in the specification in a particular order, such operations need not be performed in the particular order shown or in sequential order, and all operations need not be performed, to achieve the desirable results. Other operations that are not depicted or described can be incorporated in the example methods and processes. For example, one or more additional operations can be performed before, after, simultaneously, or between any of the described operations. Further, the operations may be rearranged or reordered in other implementations. Also, the separation of various system components in the implementations described above should not be understood as requiring such separation in all implementations, and it should be understood that the described components and systems can generally be integrated together in a single product or packaged into multiple products. Additionally, other implementations are within the scope of this disclosure.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Conjunctive language, such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Some embodiments have been described in connection with the accompanying drawings. Components can be added, removed, and/or rearranged. Orientation references such as, for example, "top" and "bottom" are for ease of ease of discussion and may be rearranged such that top features are proximate the bottom and bottom features are proximate the top. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

In summary, various embodiments and examples of compression garments and flow directing units have been disclosed. Although the systems and methods have been disclosed in the context of those embodiments and examples, it will be understood by those skilled in the art that this disclosure extends beyond the specifically disclosed embodiments to other alternative embodiments and/or other uses of the embodiments, as well as to certain modifications and equivalents thereof. This disclosure expressly contemplates that various features and aspects of the disclosed embodiments can be combined with, or substituted for, one another. Accordingly, the scope of this disclosure should not be limited by the particular disclosed embodiments described above, but should be determined only by a fair reading of the claims that follow.

## Claims

1. A garment (300) configured to be worn by a user to promote lymphatic flow, the garment comprising:
a plurality of flow directing units (200), each of the plurality of flow directing units comprising a wall (208) and a plurality of teeth (202) extending from the wall, wherein the plurality of teeth are configured to face skin of the user when the garment is worn, and wherein each of the plurality of teeth comprises a center axis (212) extending therethrough at an acute angle (222) relative to a longitudinal axis (210) of the wall so as to promote a flow of fluid within the user in a predetermined direction (206) when a compressive force is applied to the wall.

2. The garment of Claim 1, wherein the plurality of teeth are in a repeating pattern.

3. The garment of Claim 1, wherein the plurality of teeth are in a repeating saw toothed pattern.

4. The garment of Claim 1, wherein the plurality of teeth are evenly distributed along the wall.

5. The garment of any of the preceding claims, wherein the plurality of teeth include rounded ends.

6. The garment of Claim 5, wherein the plurality of teeth have a thickness that tapers to an end (216).

7. The garment of Claim 1, wherein the plurality of teeth have a thickness that is consistent.

8. The garment of Claim 1, wherein the garment comprises channels (302) configured to receive the plurality of flow directing units to maintain an orientation of the plurality of flow directing units.

9. The garment of Claim 1, wherein the garment is configured to position the plurality of flow directing units to map to lymphatic vessels of the user.

10. The garment of Claim 1, further comprising a bioimpedance sensing device (400) configured to detect a volume of a region of a body (100) of the user.

11. The garment of Claim 10, wherein the bioimpedance sensing device is configured to send and receive an electrical signal across the garment through a region of the body of the user.

12. The garment of Claim 1, further comprising one or more vibration units (500) configured to vibrate to promote lymphatic flow.

13. The garment of Claim 12, wherein the garment is configured to position the one or more vibration units at or proximate lymph nodes of the user.

## Patentansprüche

1. Kleidungsstück (300), das dazu konfiguriert ist, von einem Benutzer zur Förderung des Lymphflusses getragen zu werden, wobei das Kleidungsstück umfasst:
eine Mehrzahl von Flussleiteinheiten (200), wobei jede der Mehrzahl von Flussleiteinheiten eine Wand (208) und eine Mehrzahl von Zähnen (202) umfasst, die sich von der Wand aus erstrecken, wobei die Mehrzahl von Zähnen so konfiguriert ist, dass sie der Haut des Benutzers zugewandt sind, wenn das Kleidungsstück getragen wird, und wobei jeder der Mehrzahl von Zähnen eine Mittelachse (212) umfasst, die sich durch diesen in einem spitzen Winkel (222) relativ zu einer Längsachse (210) der Wand erstreckt, um einen Flüssigkeitsfluss innerhalb des Benutzers in einer vorgegebenen Richtung (206) zu fördern, wenn eine Druckkraft auf die Wand ausgeübt wird.

2. Kleidungsstück nach Anspruch 1, bei dem die Mehrzahl von Zähnen in einem sich wiederholenden Muster angeordnet ist.

3. Kleidungsstück nach Anspruch 1, bei dem die Mehrzahl von Zähnen in einem sich wiederholenden Sägezahnmuster angeordnet ist.

4. Kleidungsstück nach Anspruch 1, bei dem die Mehrzahl von Zähnen gleichmäßig entlang der Wand verteilt ist.

5. Kleidungsstück nach einem der vorstehenden Ansprüche, bei dem die Mehrzahl von Zähnen abgerundete Enden aufweist.

6. Kleidungsstück nach Anspruch 5, bei dem die Mehrzahl von Zähnen eine Dicke aufweist, die sich zu einem Ende (216) hin verjüngt.

7. Kleidungsstück nach Anspruch 1, bei dem die Mehrzahl von Zähnen eine einheitliche Dicke aufweist.

8. Kleidungsstück nach Anspruch 1, wobei das Kleidungsstück Kanäle (302) umfasst, die konfiguriert sind, um die Mehrzahl von Flussleiteinheiten aufzunehmen, um eine Ausrichtung der Mehrzahl von Flussleiteinheiten aufrechtzuerhalten.

9. Kleidungsstück nach Anspruch 1, wobei das Kleidungsstück konfiguriert ist, um die Mehrzahl von Flussleiteinheiten so zu positionieren, dass sie die Lymphgefäße des Benutzers abbilden.

10. Kleidungsstück nach Anspruch 1, das ferner eine Bioimpedanz-Sensoreinrichtung (400) umfasst, die konfiguriert ist, um ein Volumen einer Körperregion (100) des Benutzers zu erfassen.

11. Kleidungsstück nach Anspruch 10, bei dem die Bioimpedanz-Sensoreinrichtung konfiguriert ist, um ein elektrisches Signal über das Kleidungsstück durch einen Bereich des Körpers des Benutzers zu senden und zu empfangen.

12. Kleidungsstück nach Anspruch 1, das ferner eine oder mehrere Vibrationseinheiten (500) umfasst, die konfiguriert sind, um zur Förderung des Lymphflusses zu vibrieren.

13. Kleidungsstück nach Anspruch 12, wobei das Kleidungsstück konfiguriert ist, um die ein oder mehreren Vibrationsvorrichtungen an oder in der Nähe der Lymphknoten des Benutzers zu positionieren.

## Revendications

1. Vêtement (300) configuré pour être porté par un utilisateur afin de favoriser le flux lymphatique, le vêtement comprenant :
une pluralité d'unités de direction de flux (200), chacune de la pluralité d'unités de direction de flux comprenant une paroi (208) et une pluralité de dents (202) s'étendant à partir de la paroi, dans lequel la pluralité de dents sont configurées pour faire face à la peau de l'utilisateur lorsque le vêtement est porté, et dans lequel chacune de la pluralité de dents comprend un axe central (212) s'étendant à travers celle-ci selon un angle aigu (222) par rapport à un axe longitudinal (210) de la paroi de manière à favoriser un écoulement de fluide à l'intérieur de l'utilisateur dans une direction prédéterminée (206) lorsqu'une force de compression est appliquée à la paroi.

2. Vêtement selon la revendication 1, dans lequel la pluralité de dents sont dans un motif répétitif.

3. Vêtement selon la revendication 1, dans lequel la pluralité de dents sont dans un motif de dents de scie répétitif.

4. Vêtement selon la revendication 1, dans lequel la pluralité de dents sont réparties de manière uniforme le long de la paroi.

5. Vêtement selon l'une quelconque des revendications précédentes, dans lequel la pluralité de dents incluent des extrémités arrondies.

6. Vêtement selon la revendication 5, dans lequel la pluralité de dents présentent une épaisseur qui s'effile jusqu'à une extrémité (216).

7. Vêtement selon la revendication 1, dans lequel la pluralité de dents présentent une épaisseur qui est constante.

8. Vêtement selon la revendication 1, dans lequel le vêtement comprend des canaux (302) configurés pour recevoir la pluralité d'unités de direction de flux afin de maintenir une orientation de la pluralité d'unités de direction de flux.

9. Vêtement selon la revendication 1, dans lequel le vêtement est configuré afin de positionner la pluralité d'unités de direction de flux pour les mettre en correspondance avec les vaisseaux lymphatiques de l'utilisateur.

10. Vêtement selon la revendication 1, comprenant en outre un dispositif de détection de bio-impédance (400) configuré afin de détecter un volume d'une région d'un corps (100) de l'utilisateur.

11. Vêtement selon la revendication 10, dans lequel le dispositif de détection de bio-impédance est configuré afin d'envoyer et de recevoir un signal électrique à travers le vêtement à travers une région du corps de l'utilisateur.

12. Vêtement selon la revendication 1, comprenant en outre une ou plusieurs unités de vibration (500) configurées afin de vibrer pour favoriser l'écoulement lymphatique.

13. Vêtement selon la revendication 12, dans lequel le vêtement est configuré afin de positionner les une ou plusieurs unités de vibration au niveau ou à proximité des ganglions lymphatiques de l'utilisateur.
